# EUROPEAN PATENT APPLICATION

(11) **EP 0 998 923 A1**
(43) Date of publication of application: **10.05.2000**
(21) Application number: 98120596.6
(22) Date of filing: 30.10.1998
(51) Int. Cl.: A61K 31/00, A61K 31/135, A61K 31/495

(54) **Use of 5-HT7 receptor agonists for the treatment or prophylaxis of ischemias**

(71) Applicant: BASF AKTIENGESELLSCHAFT, 67056 Ludwigshafen (DE)
(72) Inventor: Szabo, Laszlo, Dr., 69221 Dossenheim (DE); Steiner, Gerd, Dr., 67281 Kirchheim (DE); Emling, Franz, Dr., 67065 Ludwigshafen (DE); Garcia-Ladona, Xavier, Dr., 76870 Kandel (DE); Wernet, Wolfgang, Dr., 67454 Hassloch (DE)

(57) **Abstract**

The use of 5-HT₇ receptor agonists for the preparation of a drug for prophylaxis or treatment of ischemias.

## Description

The invention relates to the use of agonists of the 5-HT₇ receptor to prevent or reduce brain damage caused by cerebral ischemia.

Cerebral ischemic disease, in particular ischemic stroke, remains a major medical condition for which there is no safe and effective treatment available. Stroke is caused by an acute alteration of blood flow to a region of the brain that results in the sudden loss of specific brain functions, so-called focal neurological deficits. Stroke is the third leading cause of death after cardiovascular disease and cancer.

So far there is no safe and effective therapy available to treat stroke or other ischemic conditions of the brain. The only approved stroke therapy is the recombinant tissue plasminogen activator t-PA, which may cause uncontrollable cerebral bleedings, especially if given more than three hours after the onset of stroke symptoms. There is therefore a continuing need to develop safe and effective methods of preventing, treating or ameliorating diseases caused by cerebral ischemia.

In the past ten years, pharmacological approaches focused mainly on the development of therapies related to glutamate neurotoxicity, which is the major component of ischemic neuronal death. In the clinical setting, however, all glutamate antagonists exhibited dose-limiting adverse CNS effects that led to discontinuation of several large stroke trials [CGS-19755, dextrorphan (Merck-Index 1966, No. 5496), aptiganel].

Because of their involvement in glutamatergic neurotransmission, serotoninergic compounds may offer a method to modulate glutamate neurotoxicity without evoking unacceptable side effects. However, the exact role of serotonin (5-hydroxytryptamine = 5-HT) in the pathophysiology of ischemic brain damage is poorly understood. Depending on localization and concentration, serotonin can either enhance or decrease the excitatory effects of glutamate; it is generally assumed that these distinct effects are mediated by different serotonin receptor subtypes.

Serotoninergic drugs have been shown to influence the outcome of cerebral ischemia in certain experimental models. Presumably selective agonists of 5-HT_{1A} receptors, which hyperpolarize the resting membrane potential, have been reported to protect the hippocampus against transient global ischemia in gerbils and rats, and to protect against focal ischemia in mice and rats. Treatment with drugs incorporating 5-HT₂ antagonist properties reduced neuronal injury associated with temporary global cerebral ischemia in rodents, but clinical trials with the 5-HT₂ antagonist naftidrofuryl failed to demonstrate an effect on mortality or motor function.

On the basis of receptor binding profiles, secondary messenger coupling and functional activity of different ligands, four main subgroups of 5-HT receptors (5-HT₁, 5-HT₂, 5-HT₃ and 5-HT₄) were defined. More recently molecular biological techniques have led to the identification of further 5-HT receptor subtypes (5-HT₅, 5-HT₆ and 5-HT₇ but evidence regarding the biological role of these new molecular entities remains fragmentary (for review see Jose and Hen, TIPS 16, 246-252 (1995)).

Stam et al. (FEBS Letters 413, 489-494 (1997)) disclose the cloning of two variants of the human serotonin 5-HT₇ receptor. They also mention that these 5-HT₇ variants are localized in brain regions thought to be implicated in depression.

The object of the invention is the use of 5-HT₇ receptor agonists for the preparation of drugs, especially for the prophylaxis and treatment of ischemias and diseases resulting from ischemias.

5-HT₇ receptor agonists mean compounds which bind to a human 5-HT₇ receptor and which mimic the effect of serotonin with this receptor. Preferred 5-HT₇ receptor agonists have an affinity to a human 5-HT₇ receptor with a Kᵢ value of 50 nmol/l or less, especially preferred 10 nmol/l or less.

Useful 5-HT₇ receptor agonists are such compounds which bind with a lower Kᵢ to a human 5-HT₇ receptor than to other human 5-HT receptors. In a preferred embodiment the Kᵢ value for human 5-HT₇ is 1/10 or less than the Kᵢ value for other human 5-HT-receptors. Such compounds are referred to as specific 5-HT₇ receptor agonists.

The affinity of a compound to a human 5-HT receptor can be measured and calculated easily by methods known to a skilled person.

Ischemias mean diseases caused by focal cerebral ischemia, such as vascular stroke, or by global cerebral ischemia, such as in with cardiac arrest, and ischemic brain damage, as well as secondary cerebral ischemia, such as in brain and spinal cord trauma.

These and other objects of the invention are provided by one or more of the embodiments described below.
In one embodiment of the invention, a method of preventing or treating diseases caused by cerebral ischemia, such as vascular stroke or cerebral ischemia due to cardiac arrest, is provided, which method comprises administering to a human patient in need thereof a therapeutically effective amount of a full or partial 5-HT₇ receptor agonist.

In another embodiment of the invention, a method of treating secondary ischemic damage associated with brain and spinal cord trauma is provided, which method comprises administering to a human patient in need thereof a therapeutically effective amount of a full or partial 5-HT₇ receptor agonist.

Thus the present invention provides the art with methods and formulations for treating diseases associated with primary or secondary cerebral ischemia, as exemplified by vascular stroke, cardiac arrest, and brain and spinal cord trauma.

A substantial amount of data supporting the role of 5-HT_{1A} receptor agonists in neuroprotection was based on experiments performed with the presumably selective 5-HT_{1A} receptor agonist 8-hydroxy-di-n-propylamino tetralin (8-OH-DPAT). After the recent identification of the 5-HT₇ receptor, it was found that 8-OH-DPAT is in fact a mixed 5-HT_{1A}/5-HT₇ agonist. Given this new knowledge about 8-OH-DPAT, in vivo experiments were devised to elaborate the 5-HT_{1A} agonist hypothesis of ischemic neuroprotection and to test the potential role of 5-HT₇ receptors in cerebral ischemia.

### Example 1

In particular, the efficacy of R(+)-8-OH-DPAT, which is the more potent enantiomer of 8-OH-DPAT, was investigated in a rat model of experimental stroke both as a monotherapy and in combination with the selective high affinity 5-HT_{1A} receptor antagonist WAY 100,635. The experiments were carried out in male Long Evans rats. Under halothane anesthesia supplemented with nitrous oxide, the right middle cerebral artery (MCA) was dissected and permanently ligated at a distal position as described in the literature. The outcome of the experiments was quantified by determining the resulting infarct volume 22 hours after MCA occlusion.

In the first series of experiments, animals were treated with 2 mg/kg R(+)-8-OH-DPAT i.v. followed by a maintenance infusion of 1 mg/kg/h R(+)-8-OH-DPAT i.v. Drug administration was started 90 min after MCA occlusion. In the second series of experiments, rats were pretreated with 2.14 mg/kg/h WAY 100,635 i.p., started 2 hours before MCA occlusion; administration of R(+)-8-OH-DPAT was subsequently performed as in the first study. Accordingly, in the second series a total dose of 2.14 mg/kg/h of WAY 100,635 had been administered for 3.5 h (total dosage = 7.49 mg/kg) before treatment with the agonist R(+)-8-OH-DPAT commenced. The blockade of 5-HT_{1A} receptors under these conditions was confirmed by the complete lack of stereotyped behavior observed in animals treated with R(+)-8-OH-DPAT alone. Under the conditions of the second series, any pharmacological effect of R(+)-8-OH-DPAT treatment are mediated by the 5-HT₇ receptor. The results are summarized in Table 1.

**Table 1**

| Treatment | Infarct volume, % of control, mean ± SD, (n) | | P, Student's t-test, two-sided | Stereotyped behavior |
|---|---|---|---|---|
| | Placebo | Treatment | | |
| R(+)-8-OH-DPAT | 100±11(12) | 72±32(12) | 0.0086 | Yes |
| WAY 100,635 + R(+)-8-OH-DPAT | 100±21(7) | 67±32(8) | 0.0378 | No |

As shown in Table 1, the administration of R(+)-8-OH-DPAT alone resulted in a statistically significant neuroprotection reflected in a reduction of infarct volume by 28%. When R(+)-8-oH-DPAT was given in combination with WAY 100,635, infarct volume was reduced by essentially the same proportion, i.e., by 33%. These results clearly demonstrate that the neuroprotective effect observed after the administration of R(+)-8-OH-DPAT is not mediated by 5-HT_{1A} receptors because the simultaneous blockade of these receptors with the highly selective 5-HT_{1A} antagonist WAY 100,635 did not change the compound's neuroprotective efficacy. Given the fact that the only other known pharmacological property of R(+)-8-OH-DPAT is its agonism at the 5-HT₇ receptor, the experiments described above clearly demonstrate that the efficacy of R(+)-8-OH-DPAT actually arises from its affinity to the 5-HT₇ receptor.

This finding shows that agonists at the 5-HT₇ receptor mediate substantial ischemic neuroprotection and that such compounds can be used for the prevention and treatment of ischemic neurological disorders, such as vascular stroke, or other diseases resulting from primary or secondary cerebral ischemia.

In addition, agonism at the 5-HT₇ receptor is not associated with any behavioral effects characteristic of 5-HT_{1A} agonists or glutamate antagonists, and may therefore represent a novel therapy with substantially reduced CNS adverse effects.

To corroborate the above finding, analogous experiments were carried out with the selective 5-HT₇ agonist Compound A which has a high affinity to the 5-HT₇ receptor (Kᵢ = 7 nmol/l) while it is only a weak agonist at the 5-HT_{1A} receptor (Kᵢ = 130 nmol/l); i.e. it exhibits approximately 19-fold selectivity for the 5-HT₇ receptor.

### Example 2

The experiments were carried out under the same conditions as described in Example 1,

A preferred 5-HT₇ receptor agonist is 1-[3-[4-[3-(trifluoromethyl)-phenyl]-piperazine-1-yl]-propyl]-3-hydroxy-1H-pyridine-2-on (= Compound A), a compound which can be prepared as discribed in example 3. The 5-HT₇ agonist Compound A was administered as an intravenous bolus followed by a maintenance infusion. The compound's effect on cerebral infarct volume is summarized in Table 2.

**Table 2**

| Dose, mg/kg + mg/kg/h i.v. | Infarct volume, % of control, mean ± SD, (n) | | P, Student's t-test, two-sided | Stereotyped behavior |
|---|---|---|---|---|
| | Placebo | Treatment | | |
| 4+2 | 100±14(10) | 78±23(10) | 0.0193 | No |

The data shown above clearly demonstrate the efficacy of A in experimental stroke and are in agreement with a neuroprotective mechanism mediated by the 5-HT₇ receptor.

Agonists of the 5-HT₇ receptor are compounds which either compete for the serotonin binding site of the receptor and possess intrinsic activity or enhance the function of the receptor by interaction with a modulatory site. Agonists in the above sense include both partial and full agonists.

The dosage and length of treatment with 5-HT₇ agonists depends on the disease state being treated. The duration of treatment may be a day, a week or longer and may, as a prophylactic treatment, last over the entire lifetime of the patient. The agonists are administered in a therapeutically effective amount; a typical human dosage of a 5-HT₇ receptor agonist ranging from about 0.01 mg/kg of body weight to about 10 mg/kg, in single or repeated doses. The dosage will vary depending on the 5-HT₇ agonist used and its relative potency. Dosage and length of treatment are readily determinable by the skilled practitioner based on the condition and stage of disease.

In therapeutic use, 5-HT₇ agonists may be administered by any route by which drugs are conventionally administered. Such routes of administration include intraperitoneal, intravenous, intramuscular, subcutaneous, intrathecal, intraventricular, as well as oral.

The effective compounds can be administered in solid or liquid form in the conventional pharmaceutical administration forms, e.g. as tablets, suppositories, solutions, ointments, creams or sprays. These are prepared in a customary manner. The active compounds can in this case be processed with the customary pharmaceutical auxiliaries such as tablet binders, fillers, preservatives, tablet disintegrants, flow-regulating agents, plasticizers, wetting agents, dispersants, emulsifiers, solvents, release-delaying agents, antioxidants and/or propellants (cf. H. Sucker et al.: Pharmazeutische Technologie [Pharmaceutical Technology], Thieme-Verlag, Stuttgart, 1991). The application forms thus obtained normally contain the active compound in an amount from 0.1 to 90% by weight.

### Example 3

### Preparation of 1-[3-[4-[3-(trifluoro-methyl)-phenyl]-piperazine-1-yl]-propyl]-3-hydroxy-1H-pyridine-2-on (= Compound A)

1.1 g (9.8 mM) of 2,3 dihydroxy-pyridine and 1.35 g (9.8 mM) of finely powdered potassium carbonate were added to 3.0 g (9.8 mM) of 1-(3-chloropropyl)-4-(3-trifluoromethyl)-phenyl-piperazine in 40 ml of dimethyl-formamide and the reaction mixture was stirred for 1 hour at 100°C. After the mixture had cooled, it was partitioned between methyl-t-butyl ether and water, the pH was adjusted to 9 and the aqueous phase was extracted with methyl-t-butyl ether. The combined organic phases were dried with sodium sulfate and evaporated down. The crude product (4.0 g) was purified by column chromatography (silica gel; developer: 3:1 mixture of ethyl acetate/ethanol).

1.8 g (48%) of product was isolated and converted into the hydrochloride with a melting point of 170 - 172°C in ethanolic solution with ethereal HCl.

## Claims

1. The use of 5-HT₇ receptor agonists for the preparation of drugs.

2. The use of 5-HT₇ receptor agonists for the preparation of drugs for treatment or prevention of ischemias.

3. The use according to claim 2 where the ischemia is stroke.

4. The use according to claim 1 to 3 where the 5-HT₇ receptor agonist is a specific 5-HT₇ receptor agonist.

5. Pharmaceutical composition comprising as effective compound a 5-HT₇ receptor agonist.

6. Pharmaceutical composition according to claim 5 for the prophylaxis or treatment of ischemic diseases.

7. Pharmaceutical compositions according to claim 6 where the ischemic disease is stroke.
